(19) Europäisches Patentamt
European Patent Office
Office européen des brevets

(11) Numéro de publication: **0 249 530**
**B1**

(12) **FASCICULE DE BREVET EUROPEEN**

(45) Date de publication du fascicule du brevet: **29.08.90**

(51) Int. Cl.⁵: **C07C 45/71**, C07C 47/277, C07C 47/198, C07D 319/06

(21) Numéro de dépôt: **87401227.1**

(22) Date de dépôt: **02.06.87**

(54) **Procédé de préparation de monoacétals du glyoxal.**

(30) Priorité: **03.06.86 FR 8607957**
**29.04.87 FR 8706106**

(43) Date de publication de la demande:
**16.12.87 Bulletin 87/51**

(45) Mention de la délivrance du brevet:
**29.08.90 Bulletin 90/35**

(84) Etats contractants désignés:
**AT BE CH DE ES FR GB GR IT LI LU NL SE**

(56) Documents cités:
**AT-B- 379 799**
**DE-A- 2 514 001**
**US-A- 2 116 016**

**JOURNAL OF ORGANICAL CHEMISTRY, vol. 38, no. 3, 1973, pages 556-560; J.M. KLIEGMAN et al.: "Glyoxal derivatives. V. Reaction of alcohols with glyoxal"**

(73) Titulaire: **SOCIETE FRANCAISE HOECHST Société anonyme dite:, 3, avenue du Général de Gaulle, F-92800 Puteaux(FR)**

(72) Inventeur: **Blanc, Alain, 21bis, rue Galvanis, F-75017 Paris(FR)**
Inventeur: **Hamedi-Sangsari, Farid, 341 rue Piemente, F-69009 Lyon(FR)**
Inventeur: **Chastrette, Francine, 22 Chemin de la Combe Martin, F-69300 Caluire(FR)**

(74) Mandataire: **Rinuy, Santarelli, 14, avenue de la Grande Armée, F-75017 Paris(FR)**

**EP 0 249 530 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de glyoxal monoacétalisé.

La présente invention concerne un procédé de préparation de glyoxal monoacétalisé de formule générale I:

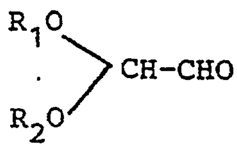

(I)

dans laquelle soit $R_1$ et $R_2$, identiques, représentent un groupement $-CHRR_3$ dans lequel R et $R_3$, identiques ou différents représentent un groupement alkyle, alcényle ou aralkyle, soit $R_1$ et $R_2$, identiques, représentent un groupement $-CH_2R$ dans lequel R représente un atome d'hydrogène, un radical alkyle, alcényle ou aralkyle, soit $R_1$ et $R_2$ forment ensemble un radical $-CH_2-(CRR)_n-CHR-$ dans lequel n représente 0 ou 1 et R conserve la signification donnée précédemment.

Comme exemples de radical $-CH_2-(CRR)_n-CHR-$, on peut citer les groupes éthylène, propylène, triméthylène, diméthyl-2,2-propane-1,3-diyl, etc.

Les réactions du glyoxal avec les alcools ont été très étudiées et elles permettent d'accéder facilement au glyoxal bis acétal correspondant (brevet US N ° 2.194.405 ; brevet GB N ° 559.362 ; brevets français N ° 1.280.792 et 2.284.584 ; H. Fischer et al, Chem. Ber., 1926, 59, 851 ; D. H. Grangaard et al, J. Amer. Chem. Soc. 1939, 61, 428 et 755 ; M. Sprung et al, J. Amer. Chem. Soc., 1951, 73, 1884 ; H. Fiesselmann et al, Chem. Ber., 1954, 87, 906 ; U. Faas et al, Chem. Ber., 1954, 87, 1343 ; J. M. Kliegman et al, J. Org. Chem., 1972, 37, 1276 ; ibid 1973, 38, 556 ; ibid 1974, 39, 1172 ; F. Chastrette et al, Bull. Soc. Chim. France, 1976, 601 et 613).

Ces méthodes directes n'ont pas permis d'accéder aux monoacétals du glyoxal, si bien que pour les obtenir, on a eu recours à des méthodes indirectes telles que l'ozonolyse de l'acétal correspondant de l'acroléine ou la coupure oxydante d'un acétal α, βdyhydroxylé comme le dihydroxy-2,3-tétraéthoxy-1,1,4,4 butane (C. Harries, Chem. Ber., 1903, 36, 1933 ; H. Fischer et al, Helv. Chim. Acta., 1939, 18, 514 ; L. Yanovkaya et al, Izvest, Akad. Nauk SSR, Otdel Khim. Nauk, 1963, 857 ; J. Hine et al, J. Amer. Chem. Soc.. 1972, 94, 6998 ; P. Noire, Chemical Abstracts, 1978, 89, référence 215108).

Ces procédés sont longs, onéreux et même parfois dangereux lorsqu'ils sont mis en oeuvre sur des quantités importantes (cf. brevet de la R.F.A. N ° 3.346.266).

Or la Demanderesse a découvert avec étonnement un nouveau procédé pour accéder rapidement et avec de bons rendements aux produits de formule I ci-dessus.

Ce procédé consiste à faire réagir du glyoxal en présence d'un catalyseur acide avec un excès de l'alcool correspondant de l'une des formules générale (II) $RR_3CHOH$, (III) $RCH_2OH$ ou (IV) $HOCH_2-(CRR)_n-CHROH$, dans lesquelles R, $R_3$ et n conservent la signification donnée précédemment, puis à arrêter la réaction dès que la concentration en monoacétal cherché de formule générale (I) décroît dans le milieu réactionnel au profit du bisacétal. La Demanderesse s'est en effet aperçue que le glyoxal, même en solution aqueuse, réagissait rapidement avec les alcools de formule générale (III) ou (IV) pour donner le monoacétal de formule générale (I), qui se transformait ensuite moins rapidement en bisacétal.

Quoique les alcools secondaires de formule générale (II) soient beaucoup moins réactifs que les alcools primaires ou les diols biprimaires ou primaires-secondaires de formule générale (III) ou (IV), on peut, en augmentant des durées réactionnelles, les faire réagir avec un rendement satisfaisant avec le glyoxal pour obtenir le mono acétal du glyoxal correspondant cherché.

Dans les très nombreux travaux consacrés à la condensation des alcools avec le glyoxal, jamais cette dissociation dans la formation du mono- et du bisacétal du glyoxal n'avait été prévue, entrevue, signalée ou obtenue. Si bien que la simplicité du procédé selon l'invention permet d'accéder aisément non seulement aux monoacétals du glyoxal précédemment décrits mais également à de nouveaux acétals tels que le diallyloxy-2,2 éthanal, qui ne peut être préparé par les méthodes connues.

Selon le procédé de l'invention, on suit d'une part la disparition du glyoxal et d'autre part la formation des acétals par l'analyse d'échantillons prélevés régulièrement dans le milieu réactionnel.

La consommation du glyoxal est suivie, par exemple, par détermination de la soude nécessaire à sa transformation en glycolate de sodium selon la réaction de Cannizzaro. Quant aux mono- et bis acétals, ils sont dosés avantageusement par chromatographie en phase gazeuse après détermination des coefficients de réponse par la méthode classique d'étalonnage interne.

Comme toutes les réactions d'acétalisation, le procédé selon l'invention est effectué en présence d'un catalyseur acide. Parmi ces catalyseurs acides, on peut citer le chlorure d'hydrogène, l'acide sulfurique, l'acide paratoluènesulfonique, le sulfate de zirconium (IV), les résines sulfoniques échangeuses d'ions sous forme acide. Avantageusement, on utilise le sulfate de zirconium (IV) ou les résines sulfoniques échangeuses d'ions sous forme acide. On emploie habituellement 50 ± 25 mmoles du catalyseur acide par mole de glyoxal engagée. En fin de réaction, ce catalyseur acide est rapidement éliminé du milieu

réactionnel par des moyens connus en eux-mêmes, comme par filtration, neutralisation au moyen d'une base appropriée, etc.

La réaction est mise en oeuvre avec un excès d'alcool par rapport au glyoxal. Cet excès peut varier dans de grandes proportions mais habituellement on emploie 12 ± 5 moles d'alcool par mole de glyoxal. Si nécessaire, on peut opérer au sein d'un solvant organique inerte compatible avec ce type de réaction de substitution nucléophile acido-catalysée tel que l'hexane, le cyclohexane, le benzène, le toluene, les solvants chlorés : chloroforme, dichlorométhane.

Le glyoxal de départ est soit solide comme son trimère cristallisé avec deux molécules d'eau, soit en solution aqueuse. Dans ce dernier cas, on peut éliminer une partie de l'eau de dissolution par distillation azéotropique préalable.

Comme exemples d'alcools utilisés dans le procédé selon l'invention, on peut citer le méthanol, l'éthanol, le propanol, le butanol-1, l'alcool isobutylique, l'alcool phényléthylique, l'alcool allylique, l'éthylèneglycol, le propanediol-1,2, le propanediol-1,3, le diméthyl-2,2-propanediol-1,3, etc.

On conduit habituellement la réaction à la température d'ébullition du milieu réactionnel mais on peut la conduire à des températures supérieures ou inférieures à celle-ci. On travaille habituellement avec distillation azéotropique de l'eau présente et/ou formée dans le milieu réactionnel avec recyclage du distillat déshydraté par des moyens connus en eux-mêmes tels que la lixiviation à travers une substance desséchante comme le sulfate de magnésium anhydre.

La réaction est généralement conduite à pression atmosphérique bien qu'une pression supérieure ou inférieure ne soient pas nuisibles au procédé.

En fin de réaction, le monoacétal du glyoxal cherché est isolé du milieu réactionnel par des moyens connus en eux-mêmes tels que la distillation fractionnée ou la cristallisation.

Les monoacétals du glyoxal sont des synthons d'oléfinoformylation très intéressants en synthèse organique et ils sont couramment employés pour accéder notamment à certains hétérocycles (D. Soerens et al, J.Org. Chem. 1979, 44, 535).

Les exemples suivants illustrent l'invention sans toutefois la limiter.

Exemple 1

Préparation du diéthoxy-2,2 éthanal

On chauffe 90 minutes à l'ebullition un mélange de
- 81,7 g de glyoxal en solution aqueuse à 71 % en poids, soit 1 mole,
-14,5 g (40 mmoles) de sulfate de zirconium (IV) à 98 % cristallisé avec 4 moles d'eau,
- 400 g (8, 7 moles) d'éthanol.

Le milieu réactionnel refroidi est ensuite filtré (ce qui permet de récupérer la majeure partie du catalyseur utilisé) puis le filtrat est traité avec 10 g (12 mmoles) d'hydrogénocarbonate de sodium sec. Après filtration des sels minéraux et évaporation sous vide de l'éthanol excédentaire, le résidu huileux obtenu est traité avec 5 volumes d'oxyde de diéthyle afin d'éliminer les dernières traces de sels minéraux. Après filtration et évaporation sous vide de l'oxyde de diéthyle introduit, l'huile résiduelle est soumise à une distillation éclair sous un vide de 10 mbar. On recueille ainsi 122 g d'un liquide distillant entre 35 et 60°C dont l'analyse en chromatographie phase vapeur révèle la présence de 70 % environ de diéthoxy-2,2 éthanal et 30 % de tétraéthoxy-1,1,2,2 éthane.

Une deuxième distillation sur un appareil à distiller à colonne à bande tournante permet d'isoler 80 g (0,61 mole) de diéthoxy-2,2 éthanal distillant à 43 ± 2°C sous 11 mbar.

Exemple 2

Préparation du dibutoxy-2,2 éthanal

On chauffe 5 heures à l'ébullition, avec élimination de l'eau présente et formée dans le milieu réactionnel par distillation azéotropique et recyclage des solvants, un mélange de :
- 145 g (1 mole) de glyoxal en solution aqueuse à 40 % en poids,
- 740 g (10 moles) de butanol-1,
- 100 g (100 méq.) de résine sulfonique sous forme acide, IMAC C16P, commercialisée par la société AKZO (cf. Kirk-Othmer, 3ème édition, volume 13, 696),
- 860 g d'hexane.

A ce stade, une analyse par chromatographie en phase vapeur d'un échantillon du milieu réactionnel révèle la présence de 0,1 mole de glyoxal non transformé, 0,85 mole de dibutoxy-2,2 éthanal et de 0,05 mole de tétrabutoxy-1,1,2,2 éthane.

Le milieu réactionnel est refroidi, filtré puis le filtrat est traité avec 50 g (0,6 mole) d'hydrogénocarbonate de sodium. Après filtration des sels minéraux et évaporation sous vide des solvants, on obtient 250 g d'un liquide jaune clair qui est distillé sous un vide de 2 mbar. On recueille la fraction distillant à 71 ±

3°C. On obtient ainsi 122 g de dibutoxy-2,2 éthanal soit un rendement de 65 % de la théorie calculée par rapport au glyoxal engagé. En poursuivant la distillation, on recueille 9,6 g (30 mmoles) de tétrabutoxy-1,1,2,2 éthane distillant à 120 ± 5°C (Fiesselmann et al, Chem. Ber., 1954, 87, 911, $Eb_{10}$ = 160 ± 1°C).

Analyses physiques

RMN $^1H$ ($CDCl_3$)
1 ppm (massif, 6H, $CH_3$)
1,4 ppm (massif, 8H, $CH_2$-$CH_2$)
3,6 ppm (massif, 4H, -$CH_2$-O)
4,55 ppm (doublet, 1H, J = 2 Hz, >CH-C-
9,4 ppm (doublet, 1H, J = 2 Hz, CHO).

A la connaissance de la Demanderesse, ce produit n'est pas décrit dans la littérature.

Exemple 2

Préparation du diallyloxy-2,2 éthanal

On chauffe au reflux pendant t heures avec distillation azéotropique de l'eau et recyclage du solvant organique à l'aide d'une appareil Dean-Stark une solution de :
- 1 mole de glyoxal en solution aqueuse à 40 % en poids,
- 50 mmoles d'un catalyseur C (ou 50 méq. acide d'une résine sulfonique),
- 10 moles d'alcool allylique,
- 1600 cm³ d'un solvant S donnant un azéotrope avec l'eau.

Lorsque les dosages de diallyloxy-2,2 éthanal et de tétraallyloxy-1,1,2,2 ethane effectués périodiquement au cours du chauffage indiquent une décroissance de la concentration en diallyloxy-2,2 ethanal au profit du tétraallyloxy-1,1,2,2 ethane, on refroidit le milieu réactionnel puis on élimine le catalyseur par filtration et/ou par neutralisation avec un carbonate acide d'un métal alcalin suivie d'une filtration et ensuite on distille le milieu réactionnel. Après élimination des solvants, on recueille une première fraction distillant à 62 ± 2°C sous 6 mbar de p moles constituée par le produit attendu puis une deuxième fraction distillant à 100 ± 5°C sous 5 mbar de q moles constituée par le tétraallyloxy-1,1,2,2 éthane.

Le tableau I ci-après résume les essais effectués.

| Essai N° | t en heures | C catalyseur | S solvant | p | q |
|---|---|---|---|---|---|
| 1 | 2 | $H_2SO_4$ conc | benzène | 0,49 | 0,18 |
| 2 | 2 | $Zr(SO_4)_2$ | benzène | 0,51 | 0,27 |
| 3 | 5 | Résine A | benzène | 0,70 | 0,16 |
| 4 | 1,5 | Résine B | benzène | 0,54 | 0,29 |
| 5 | 5 | $Zr(SO_4)_2$ | chloroforme | 0,53 | 0,12 |
| 6 | 10 | Résine A | chloroforme | 0,70 | 0,13 |
| 7 | 6 | Résine B | chloroforme | 0,57 | 0,23 |

Résine A: résine macropore commercialisée sous al dénomination IMAC C 16 P (cf. Encyclopedia of Chemical Technology, Kirk-Othmer, 3ème edition, 13, 696).
Résine B: résine NAFION (cf. Encyclopedia of Chemical Technology, Kirk-Othmer, 3ème edition, S.559).

Analyses physiques

RMN$^1$H ($CDCl_3$)
4,2 ppm (doublet, 4H, - $OCH_2$)
4,7 ppm (doublet, 1H, J = 2 Hz, >CH-C)
5,2 ppm (massif,4H, $CH_2$)
5,8 ppm (massif, 2H, =CH-)
9,5 ppm (doublet, 1H, J = 2 Hz, CHO)

A la connaissance de la Demanderesse, ce produit n'est pas décrit dans la littérature.

Exemple 4

Préparation du diméthoxy-2,2 éthanal

On chauffe 200 minutes à l'ébullition un mélange de :
- 81,7 g de glyoxal en solution aqueuse à 71 % en poids, soit 1 mole,
- 14,5 g de sulfate de zirconium (IV),
- 400 g (12,5 moles) de méthanol.

Puis le milieu réactionnel est traité comme dans l'exemple 1. On obtient ainsi 73 g (0,7 mole) de diméthoxy-2,2 éthanal distillant sous 40 mbar à 59 ± 3°C (L.A. Yanovskaya et al, Izvest. Akad. Nauk SSR, Otdel. Khim. Nauk, 1963, 857, $Eb_{39}$ = 59 ± 1°C) et 30 g (0,2 mole) de tétraméthoxy-1,1,2,2 éthane distillant sous 12 mbar à 53 ± 2°C.

Exemple 5

On chauffe à l'ébullition pendant 4 heures un mélange de :
- 70 g de glyoxal cristallisé commercial (trimère cristallisé avec 2 moles d'eau, F = 140-150°C), soit 1 mole de glyoxal monomère,
- 14,5 g (40 mmoles) de sulfate de zirconium à 98 % cristallisé avec 4 moles d'eau,
- 740 g (10 moles) de méthyl-2 propanol-1,

puis on traite le milieu réactionnel comme dans l'exemple 1. On isole ainsi 113 g (0,6 mole) de diisobutoxy-2,2 éthanal distillant à 52°C sous 6 mbar et 95 g (0,3 mole) de tétraisobutoxy-1,1,2,2 éthane distillant à 94-99°C sous 5 mbar.

Analyses physiques

RMN$^1$H ($CDCl_3$)
0,94 ppm (doublet, 12H, $CH_3$)
1,9 ppm (massif, 2H, CH)
3,4 ppm (massif, 4H, $CH_2O$)
4,5 ppm (doublet, 1H, J = 2 Hz)
9,5 ppm (doublet, 1H, J = 2 Hz, CHO)

Exemple 6

Préparation du formyl-2 diméthyl-5,5 dioxanne-1,3

On chauffe 4 heures à l'ébullition, avec distillation azéotropique de l'eau présente et formée dans le milieu réactionnel et recyclage des solvants, une solution de :
- 145 g (1 mole) de glyoxal en solution aqueuse 35 à 40 %,
- 104 g (1 mole) de diméthyl-2,2 propanediol-1,3,
-3,8 g (0,02 mole) d'acide paratoluènesulfonique,
- 440 g de benzène.

Puis on traite le milieu réactionnel comme dans l'exemple 2.

On recueille ainsi 72,2 g (0,5 mole) de formyl-2 diméthyl-5,5 dioxanne-1,3 distillant à 81 ± 2°C sous 30 mbar.

Analyses physiques

RMN$^1$H ($CDCl_3$)
0,6 ppm (singulet, 3H, $CH_3$)
1 ppm (singulet, 3H $CH_3$)
3,5 ppm (massif, 4H, $CH_2$)
4,5 ppm (doublet, 1H, J = 52 Hz, CH)
9,3 ppm (doublet, 1H, J = 2 Hz, CHO)

Exemple 7

Préparation du diisopropoxy-2,2 éthanal

On chauffe 8 heures à l'ébullition une solution constituée par :
- 76 g de glyoxal en solution aqueuse à 76,31 % en poids, soit 1 mole,
- 420,7 g de 2-propanol, soit 7 moles,
- 19 g (0,1 mole) d'acide paratoluènesulfonique cristallisé avec une mole d'eau,
puis après refroidissement de la solution réactionnelle, on neutralise l'acide paratoluènesulfonique présent avec du bicarbonate de sodium, on filtre ensuite les sels minéraux et on élimine sous vide l'alcool isopropylique non transformé. L'huile résiduelle est ensuite distillée sous vide. On isole ainsi le diisopropoxy-2,2 éthanal distillant sous un vide de 1 mbar à 32 ± 3°C.

Le diisopropoxy-2,2 éthanal est, à la température ambiante, un liquide incolore, très fluide, soluble dans l'eau et les solvants organiques usuels.

Analyses physiques

RMN$^1$H ($CDCl_3$) à 200 MHz
9,36 ppm (d, 1H, J = 2,9 Hz, CHO)
4,61 ppm (d, 1H, J = 2,9 Hz, CH)
3,94 ppm (heptuplets, 2H, $CH(Me)_2$ J = 6 Hz)
1,27 ppm (d, 6H, Me, J = 6 Hz)
1,20 ppm (d, 6H, Me, J = 6 Hz)

Il va de soi que la présente invention n'a été décrite qu'à titre purement explicatif et nullement limitatif et que toute modification, notamment au niveau des équivalences techniques, pourra y être apportée sans sortir de son cadre.

**Revendications**

1. Procédé d'obtention des produits de formule générale (I):

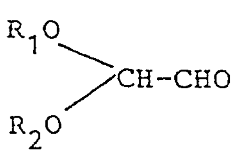

(I)

dans laquelle, soit $R_1$ et $R_2$, identiques représentent un groupement $-CHRR_3$ dans lequel R et $R_3$, identiques ou différents représentent un groupement alkyle, alcényle ou aralkyle, soit $R_1$ et $R_2$, identiques, représentent un groupement $-CH_2R$ dans lequel R représente un atome d'hydrogène, un radical alkyle, alcényle ou aralkyle, soit $R_1$ et $R_2$ forment ensemble un radical $-CH_2-(CRR)_n-CHR-$ dans lequel n représente 0 ou 1 et R conserve la signification donnée précédemment, caractérisé par le fait que l'on fait réagir du glyoxal en présence d'un catalyseur acide avec un excès de l'alcool correspondant de formule générale (II) $RR_3CHOH$, (III) $RCH_2OH$ ou (IV) $HO-(CRR)_n-CRHOH$ où R, $R_3$ et n conservent la signification donnée précédemment, puis que l'on arrête la réaction dès que la concentration en monoacétal cherché de formule générale (I) décroît dans le milieu réactionnel qu profit du bisacétal, la réaction étant suivie par analyse d'échantillons prélevés régulièrement dans le milieu réactionnel.

2. Procédé selon la revendication 1, caractérisé par le fait que le catalyseur acide est du sulfate de zirconium.

3. Procédé selon la revendication 1, caractérisé par le fait que le catalyseur acide est une résin sulfonique échangeuse d'ions sous forme acide.

4. Procédé selon l'une quelconque des revendications 1 à 3, caractérisé par le fiat que l'alcool de formule générale (II) $RR_3CHOH$ est le propanol-2.
5. Procédé selon l'une quelconque des revendications 1 à 3, caractérisé par le fait que l'alcool de formule générale (III) $RCH_2OH$ est l'alcool allylique.
6. Procédé selon l'une quelconque des revendications 1 à 3, caractérisé par le fait que l'alcool de formule générale (III) $RCH_2OH$ est le méthanol.
7. Procédé selon l'une quelconque des revendications 1 à 3, caractérisé par le fait que l'alcool de formule générale (III) $RCH_2OH$ est le butano-1.

## Claims

1. A process for the preparation of products having the general formula (I):

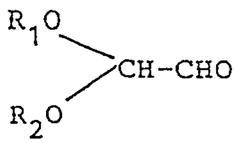

(I)

where in each of $R_1$ and $R_2$, which are identical, represents a $-CHRR_3$ group in which R and $R_3$, which are identical or different, represent an alkyl, alkenyl or aralkyl group, or $R_1$ and $R_2$, which are identical, represent a $-CH_2R$ group in which R represents a hydrogen atom, or an alkyl, alkenyl or aralkyl radical, or $R_1$ and $R_2$ together form a $-CH_2-(CRR)_n-CHR-$ radical in which n represents 0 or 1 and R has the meaning given above, characterized by the fact that glyoxal is reacted in the presence of an acid catalyst with an excess of the corresponding alcohol of general formula (II) $RR_3CHOH$, (III) $RCH_2OH$ or (IV) $HO-(CRR)_n-CHROH$, in which R, $R_3$ and n have the meanings given above, and in that the reaction is stopped as soon as the concentration in the reaction medium of the desired monoacetal of general formula (I) decreases in favour of the bisacetal, progress of the reaction being monitored by analysis of samples taken at regular intervals from the raction medium.
2. A process as claimed in claim 1, characterized by the fact that the acid catalyst is zirconium sulphate.
3. A process as claimed in claim 1, characterized by the fact that the acid catalyst is a sulphonic ionexchange resin in acid form.
4. A process as claimed in any one of claims 1 to 3, characterized by the fact that the alcohol of general formula (II) $RR_3CHOH$ is propan-2-ol.
5. A process as claimed in any one of claims 1 to 3, characterized by the fact that the alcohol of general formula (III) $RCH_2OH$ is allyl alcohol.
6. A process as claimed in any one of claims 1 to 3, characterized by the fact that the alcohol of general formula (III) $RCH_2OH$ is methanol.
7. A process as claimed in any one of claims 1 to 3, characterized by the fact that the alcohol of general formula (III) $RCH_2OH$ is butan-1-ol.

## Patentansprüche

1. Verfahren zur Herstellung von Produkten der allgemeinen Formel (I)

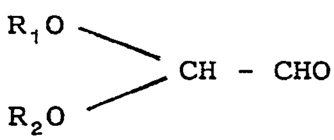

(I),

worin entweder $R_1$ und $R_2$ identisch sind und eine Gruppe $-CHRR_3$ bedeuten, wobei R und $R_3$, die gleich oder verschieden sind, eine Alkyl-, Alkenyl- oder Aralkylgruppe darstellen, oder $R_1$ und $R_2$ identisch sind und eine Gruppe $-CH_2R$ bedeuten, wobei R ein Wasserstoffatom, einen Alkyl-, Alkenyl- oder Aralkylrest darstellt, oder $R_1$ und $R_2$ miteinander einen Rest $-CH_2-(CRR)_n-CHR$ bilden, in wechem n Null oder 1 ist und R die oben angegebene Bedeutung hat, dadurch gekennzeichnet, daß man Glyoxal in Anwesenheit eines sauren Katalysators mit einem Überschluß eines Alkohols der allgemeinen Formel
$RR_3CHOH$ (II), $RCH_2OH$ (III) oder $HO-(CRR)_n-CRHOH$ (IV),
worin R, $R_3$ und n die oben angegebene Bedeutung haben, umsetzt und dann die Reaktion abbricht, sobald die Konzentration an gewünschtem Monoacetal der allgemeinen Formel (I) im Reaktionsmedium zugunsten des Diacetals nachläßt, wobei die Reaktion durch Analyse von regelmäßig aus dem Reaktionsmedium entnommenen Proben überwacht wird.
2. Verfahren nach Anspruch 1, dadurch gekennzeichnet, daß der saure Katalysator Zirkoniumsulfat ist.

3. Verfahren nach Anspruch 1, dadurch gekennzeichnet, daß der saure Katalysator ein Ionenaustauschersulfonsäureharz in saurer Form ist.

4. Verfahren nach einem der Ansprüche 1 bis 3, dadurch gekennzeichnet, daß der Alkohol der allgemeinen Formel $RR_3CHOH$ (II) Propanol-2 ist.

5. Verfahren nach einem der Ansprüche 1 bis 3, dadurch gekennzeichnet, daß der Alkohol der allgemeinen Formel $RCH_2OH$ (III) Allylalkohol ist.

6. Verfahren nach einem der Ansprüche 1 bis 3, dadurch gekennzeichnet, daß der Alkohol der allgemeinen Formel (III) $RCH_2OH$ Methanol ist.

7. Verfahren nach einem der Ansprüche 1 bis 3, dadurch gekennzeichnet, daß der Alkohol der allgemeinen Formel $RCH_2OH$ (III) Butanol-1 ist.